**Europäisches Patentamt**

**European Patent Office**

**Office européen des brevets**

(19)

(11) Veröffentlichungsnummer: **0 017 096**
**B1**

(12) **EUROPÄISCHE PATENTSCHRIFT**

(45) Veröffentlichungstag der Patentschrift:
**21.04.82**

(51) Int. Cl.³: **G 01 N 33/34**, G 01 N 33/26

(21) Anmeldenummer: **80101473.9**

(22) Anmeldetag: **20.03.80**

(54) **Verfahren und Vorrichtung zur Prüfung des Durchschlagverhaltens von Beschichtungsmassen und Beschichtungsträgern.**

(30) Priorität: **24.03.79 DE 2911649**

(43) Veröffentlichungstag der Anmeldung:
**15.10.80 Patentblatt 80/21**

(45) Bekanntmachung des Hinweises auf die Patenterteilung:
**21.04.82 Patentblatt 82/16**

(84) Benannte Vertragsstaaten:
**AT BE FR GB IT NL SE**

(56) Entgegenhaltungen:
**DE-A-2 003 758**
**DE-B-1 648 729**
**US-A-2 353 852**
**US-A-3 512 003**

(73) Patentinhaber: **Feldmühle Aktiengesellschaft,
Fritz-Vomfelde-Platz 4, D-4000 Düsseldorf 11 (DE)**

(72) Erfinder: **Bergmann, Kurt Werner, Dr. Dipl.-Chem.,
Sperbergweg 7, D-4057 Brüggen 1 (DE)**
Erfinder: **Westerhuis, Popko Julius, Schellingstrasse 24,
D-5142 Hückelhoven-Baal (DE)**

(74) Vertreter: **Uhlmann, Hans, Dr. rer.nat., Dipl.-Chem.,
Gladbacher Strasse 189, D-4060 Viersen 1 (DE)**

## Verfahren und Vorrichtung zur Prüfung des Durchschlagverhaltens von Beschichtungsmassen und Beschichtungsträgern

Die vorliegende Erfindung betrifft ein Verfahren und eine Vorrichtung zur Prüfung des Durchschlagverhaltens von Beschichtungsmassen und/oder für die Beschichtung vorgesehenen, bandförmigen Beschichtungsträgern, bei dem ein Beschichtungsträger mit einer Beschichtungsmasse beaufschlagt wird.

Flächige Beschichtungsträger, wie Papier, Karton, Vliesstoff oder Gewebe werden in großem Umfang mit Beschichtungen versehen. Die Beschichtung dient dabei der Verbesserung und Gestaltung der Oberfläche und soll das Substrat nur so weit penetrieren, daß eine gute Verbindung zwischen Beschichtung und Substrat resultiert. Manchmal kommt es jedoch während des Beschichtungsprozesses an einigen Stellen des Beschichtungsträgers zur vollkommenen Penetration. Insbesondere bei schnellaufenden Maschinen werden durch dieses als Durchschlagen bezeichnete Verhalten erhebliche Betriebsstörungen verursacht, weil die durchgeschlagene Beschichtungsmasse sich auf den unterhalb der Warenbahn angeordneten Maschinenelementen, insbesondere auf den Gegenwalzen, absetzt und dort aufbaut. Die Folge sind Beschichtungsfehler und Abrisse der Bahn. Besonders störanfällig sind in dieser Hinsicht die schnellaufenden Streichmaschinen der Papierindustrie, insbesondere diejenigen, auf denen leichtgewichtige Papiere beschichtet werden. Solche, allgemein unter der Bezeichnung LWC-Papiere bekannten Papiere haben als Rohpapier ein Flächengewicht zwischen 36 und 40 g/m². Die Beschichtung erfolgt aus wäßrigen Beschichtungsmassen mit Feststoffgehalten zwischen 45 und 70 Gew.-%. Zumeist werden 8 bis 12 g/m² und Seite Trockensubstanz aufgetragen. Das aus wirtschaftlichen Gesichtspunkten extrem niedrig eingestellte Flächengewicht des Streichrohpapiers verursacht dabei enorme Schwierigkeiten während des Beschichtungsprozesses, insbesondere, wenn solche Beschichtungsmassen verarbeitet werden, die im unteren Bereich der obengenannten Grenzen für den Feststoffgehalt liegen. Man ist deshalb ständig bemüht, durch geeignete Maßnahmen ein Durchschlagen der Streichmasse auf die unter der Warenbahn und dem Rakel angeordnete Coater-Walze zu verhindern.

Es erweist sich dabei als schwerwiegender Nachteil, insbesondere bei Neuentwicklungen und Rezepturänderungen, daß bisher eine zuverlässige, im Labormaßstab durchführbare Prüfmethode fehlt, die eine Voraussage über die Eignung von Beschichtungsmassen und -trägern hinsichtlich ihres Durchschlagverhaltens unter Produktionsbedingungen erlaubt.

In der Vergangenheit sind zwar bereits verschiedene Laborbeschichtungsverfahren und entsprechende Vorrichtungen vorgeschlagen worden, ohne daß jedoch das anstehende Problem gelöst wurde.

Ein in der US-A-2 353 852 beschriebenes Prüfverfahren verwendet eine Beschichtungsvorrichtung, bei der Proben unter Anwendung von geringem Druck mit einem dünnen Oberflächenfilm beschichtet werden. Diese Prüfmethode dient der Beurteilung der Gleichmäßigkeit der Oberfläche, wodurch eine Aussage über das Durchschlagverhalten jedoch noch nicht ermöglicht wird.

Ferner ist aus der DE-A-2 003 758 eine Labor-Sizepresse bekannt, die zur Ermittlung der Adsorption von Flüssigkeiten durch bahn- oder blattförmige Materialien verwendet wird. Dabei werden die imprägnierten Proben ausgewogen.

In Ermangelung einer geeigneten Prüfmethode, die das Zusammenwirken zwischen Beschichtungsträger und Beschichtungsmasse erfaßt, behilft man sich in der Praxis des Papierstreichens bisher damit, gewisse Parameter der Streichmasse, die für das Durchschlagen als relevant gelten, zu messen und dadurch zu einer Aussage über das Durchschlagverhalten zu kommen. Es hat sich jedoch gezeigt, daß diese Einzelmessungen in den meisten Fällen von geringem Wert sind.

Auch weitere das Durchschlagverhalten bestimmende Faktoren, wie z. B. das Flächengewicht des Streichrohpapiers, unterliegen ständiger Kontrolle, da mit sinkendem Flächengewicht in erhöhtem Maße kleine Löcher entstehen können, die das Durchschlagen begünstigen. Trotzdem kommt es im Produktionsbetrieb immer wieder zu dem gefürchteten Durchschlagen und den damit verbundenen Maschinenstillständen, da selbst kleine, sich der bisherigen Meßtechnik entziehende Abweichungen von Beschichtungsmasse oder Beschichtungsträger die Ursache zu häufig wiederkehrenden Betriebsstörungen bilden können.

Die bekannten Verfahren und Vorrichtungen sind jedoch noch nicht zur Lösung der Aufgabe geeignet, die der vorliegenden Erfindung zugrunde liegt und die darin besteht, ein Verfahren und eine Vorrichtung zur Prüfung des Durchschlagverhaltens von Beschichtungsmassen und/oder Beschichtungsträgern zu entwickeln, d. h., die stellenweise erfolgende Penetration des Beschichtungsträgers durch die Beschichtungsmasse festzustellen. Insbesondere will die vorliegende Erfindung eine Prüfmethode schaffen, die mit geringem zeitlichem und apparativem Aufwand unter praxisnahen Bedingungen arbeitet und dabei aus dem direkten Zusammenwirken von Beschichtungsmassen und Beschichtungsträgern sichere Rückschlüsse auf das Durchschlagverhalten unter Produktionsbedingungen erlaubt.

Zur Lösung dieser Aufgabe sieht die Erfindung ein Verfahren vor, daß dadurch gekennzeichnet ist, daß eine Standardbeschichtungsmasse oder eine zu prüfende Beschichtungsmasse unter Anwendung von Scherung und Druck durch eine zu prüfende Bahn des Beschichtungsträgers oder durch eine ein bekanntes Durchschlagverhalten aufweisende Standardprüfbahn auf ein unter dem Beschichtungsträger angeordnetes, flächiges Kontrastmaterial gepreßt wird, das in seiner Farbe von der Beschichtungsmasse abweicht und daß die auf das Kontrastmaterial durchgedruckten Markierungen der Beschichtungsmasse als Maß für das Durchschlagverhalten der Beschichtungsmasse bzw. des Beschichtungsträgers dienen.

Das erfindungsgemäße Verfahren zeichnet sich dadurch aus, daß in bisher nicht bekannter Weise das Durchschlagverhalten von Beschichtungsmasse und Beschichtungsträgern im direkten Zusammenspiel dieser beiden Komponenten ermittelt wird. Die bisherige Praxis, das Durchschlagverhalten an Hand von Einzelfaktoren, die an den Beschichtungsträgern bzw. Beschichtungsmassen gemessen wurden, in mehr oder weniger genauer Weise abzuschätzen, kann damit aufgegeben werden. Insbesondere bei Neuentwicklungen von Beschichtungsmassen und -trägern bietet das erfindungsgemäße Verfahren den Vorteil, mit einem minimalen Aufwand an Zeit und Untersuchungsmaterial ein aussagekräftiges Untersuchungsergebnis zu erzielen.

Zur Untersuchung einer Beschichtungsmasse wird dabei eine standardisierte Prüfbahn verwendet, während man zur Untersuchung eines Beschichtungsträgers eine in ihrem Durchschlagverhalten bereits bekannte Beschichtungsmasse heranzieht.

Das erfindungsgemäße Verfahren ermöglicht dabei durch unterschiedliches Anlegen der Beschichtungsträgerbahn das Durchschlagverhalten sowohl von deren Ober- als auch von deren Unterseite aus festzustellen. Selbstverständlich können sowohl neue Beschichtungsmassen als auch neue Beschichtungsträger in ihrem Zusammenwirken untersucht und aufeinander abgestimmt werden.

Die durch Druckanwendung durch die porenartigen Öffnungen des Beschichtungsträgers gepreßte Beschichtungsmasse, die z. B. von weißer Farbe ist, zeichnet sich auf dem unter dem Beschichtungsträger angeordneten Kontrastmaterial ab, das beispielsweise eine schwarze Färbung aufweist. Das Durchschlagverhalten ist um so größer, je mehr als Markierungen bezeichnete Farbflecken der Beschichtungsmasse auf dem Kontrastmaterial sichtbar werden bzw. je größer diese sind.

Als geeignete Kontrastmaterialien können Papier, Karton, Vliesstoff oder Folien verwendet werden, wobei Papiere besonders bevorzugt sind.

Um ein Verlaufen der Beschichtungsmassen zu verhindern und möglichst scharfe Markierungen zu erhalten, hat es sich als zweckmäßig erwiesen, Kontrastmaterialien mit relativ rauher Oberfläche zu verwenden, die zudem noch ein gewisses Saugvermögen für das Lösungsmittel oder Dispersionsmedium der Beschichtungsmassen aufweisen.

Besonders deutlich läßt sich das Durchschlagverhalten darstellen, wenn man die Markierungen der Beschichtungsmasse auf dem Kontrastmaterial nicht auf der gleichen Flächeneinheit erzeugt, die in ihren Maßen dem untersuchten Beschichtungsträger entspricht. Es hat sich vielmehr als vorteilhaft erwiesen, die auf einer bestimmten Testlänge des Beschichtungsträgers durchdringende Beschichtungsmasse auf einer um ein gegenüber dieser Testlänge mehrfach verkleinerten Flächeneinheit des Kontrastmaterials aufzufangen. Als geeignet haben sich dabei Verhältnisse von 3 : 1 bis 15 : 1 erwiesen, d. h., daß die auf 3 bzw. 15 m Länge des Beschichtungsträgers durchdringende Beschichtungsmasse auf einer Fläche des Kontrastmaterials aufgefangen wird, die einem umlaufenden Meter entspricht.

Um eine genaue und faltenfreie Durchführung insbesondere kurzer Bahnen zu ermöglichen, genügt es vielfach, die Prüfbahn von Hand durch den Farbsumpf zu führen. Dabei wird eine Anfangsmarkierung der Prüfbahn genau auf die Berührungslinie der beiden Quetschwalzen eingestellt, die Beschichtungsmasse eingefüllt und die Prüfbahn dann bis zur Endmarkierung durchgeführt. Da diese Endmarkierung jedoch in den Farbsumpf eintaucht und nicht mehr genau zu erkennen ist, hat es sich zu einer präzisen Versuchsdurchführung als vorteilhaft erwiesen, wenn das Ende der Prüfbahn mit einer für die Beschichtungsmasse undurchdringlichen Materialbahn verbunden ist. Diese Materialbahn kann z. B. eine Kunststoffolie sein. Bei der Untersuchung von wäßrigen Beschichtungsmassen erfolgt die Verbindung vorteilhaft mit einem Selbstklebeband, wobei das Selbstklebeband gleichzeitig die Endmarkierung darstellt. Auf diese Weise kann das Ende der Prüfbahn mit der angeklebten Folie bis unterhalb des Walzenspaltes gefahren werden, ohne daß die Gefahr besteht, daß mehr Markierungen auf dem Kontrastmaterial entstehen als der Länge der Prüfbahn entsprechen.

In einer zweckmäßigen Verfahrensvariante erfolgt die Beurteilung des Durchschlagverhaltens in einfacher Weise dadurch, daß das für die Prüfung benutzte Kontrastmaterial mit einer Skala verglichen wird, die aus Standard-Kontrastmaterial besteht, auf das Markierungen aufgebracht sind, die einem abgestuften Durchschlagverhalten entsprechen.

Eine solche Vergleichsskala erhält man, wenn man z. B. 5 oder 10 für die Prüfung benutzte Kontrastmaterialien, deren Markierungen ein unterschiedliches Durchschlagverhalten erkennen lassen, zusammenstellt. Einem Kontrastmaterial, das nur wenige und schwache Markierungen zeigt, wird dabei die Stufe 1 zugeordnet und ein Kontrastmaterial, das sehr starke Markierungen aufweist und in der Regel das Durchschlagverhalten eines Beschichtungsträgers oder einer Beschichtungsmasse kennzeichnet, das in der betrieblichen Praxis zu Betriebsstörungen führt, wird an das Ende der Skala gesetzt und z. B. mit der Nr. 10 versehen. Durch einfachen Vergleich der durch das erfindungsgemäße Verfahren erhaltenen Kontrastmaterialien mit der Bewertungsskala und Zuordnung einer entsprechenden Stufe ist nun eine sichere Kennzeichnung des Durchschlagverhaltens einer Beschichtungsmasse oder eines Beschichtungsträgers möglich.

Eine meßtechnische Auswertung der Versuchsergebnisse ist vorzugsweise durch die Verfahren der quantitativen Bildanalyse, Remissionsmessung oder Farbdichtemessung möglich. Ganz besonders bevorzugt ist hierbei die quantitative Bildanalyse, da hierbei ein z. B. über eine Fernsehkamera aufgenommenes Bild durch einen Elektronenrechner zahlenmäßig und flächenmäßig ausgewertet werden kann.

Für die Remissions- und Farbdichtemessung können die unter den Bezeichnungen Remissionsfotometer bzw. Densitometer bekannten Geräte Verwendung finden.

Eine zweckmäßige Vorrichtung zur Durchführung des Verfahrens ist dadurch gekennzeichnet, daß sie die folgenden Merkmale aufweist:

Zwei nebeneinanderliegende und gegeneinander anpreßbare Quetschwalzen, von denen eine der Quetschwalzen einen Mantel aus Beschichtungsmasse abweisendem Material aufweist oder im Bereich des Walzenspaltes mit einem die Beschichtungsmasse abweisendem und mitlaufendem Material abgedeckt ist,
ein die andere Walze zumindest im Bereich des Walzenspaltes teilweise umschlingendes Kontrastmaterial, das in seiner Farbe von der Beschichtungsmasse abweicht.

Zur Untersuchung einer Beschichtungsmasse wird eine ein bekanntes Durchschlagverhalten aufweisende Prüfbahn so angeordnet, daß sie über dem Kontrastmaterial aufliegt und zumindest teilweise die gleiche Walze wie das Kontrastmaterial umschlingt. Aus der zu untersuchenden Beschichtungsmasse wird zwischen den gegeneinander angepreßten Quetschwalzen ein Farbsumpf gebildet. Die ein bekanntes Durchschlagverhalten aufweisende Prüfbahn wird von oben nach unten so durch diesen Farbsumpf geführt, daß sie mit einer Seite im Kontakt zum Farbsumpf steht, während die andere Seite auf dem Kontrastmaterial aufliegt. Beim Durchführen durch den Farbsumpf preßt sich die Beschichtungsmasse durch die Prüfbahn und je nach Beschaffenheit der Beschichtungsmasse bzw. der Prüfbahn werden auf dem Kontrastmaterial mehr oder weniger größere oder kleinere Markierungen erzeugt.

Dem Material, aus dem der Mantel der einen Walze hergestellt ist — es ist dies die Walze, die der vom Kontrastmaterial und der Prüfbahn teilweise umschlungenen gegenüberliegt —, kommt deswegen besondere Bedeutung zu, weil durch die Wahl eines geeigneten Material ein Kleben der Prüfbahn an dieser Walze bzw. ein Zerreißen der Prüfbahn verhindert wird. Geeignet ist z. B. ein Überzug aus Polytetrafluoräthylen.

Ganz besonders bevorzugt ist jedoch eine Abdeckung aus einem die Beschichtungsmasse abweisenden, mitlaufenden Material. Solche Materialien sind z. B. die bekannten Releasepapiere.

Für die Untersuchung wäßriger Beschichtungssysteme haben sich Folienbahnen aus Kunststoff als geeignet erwiesen. Ganz besonders geeignet sind Polyäthylenfolien. Insbesondere bei Beschichtungsträgern mit geringem Flächengewicht, die in Kontakt mit dem Lösungsmittel oder dem Dispersionsmedium nur eine minimale Zugfestigkeit besitzen, ist die Verwendung eines mitlaufenden Abdeckmaterials von Vorteil, da es praktisch als Stützbahn fungiert, infolge seiner Releaseeigenschaften aber problemlos von dieser zu trennen ist.

Für eine exakte Versuchsdurchführung ist es wichtig, daß zwischen den Quetschwalzen kein Schlupf entsteht. Zweckmäßigerweise besteht deshalb zwischen beiden Quetschwalzen eine kraft- oder formschlüssige Verbindung. In einer vorteilhaften Ausführungsform erfolgt dabei die Übertragung des Antriebs über Zahnräder oder eine Riemenverbindung von einer durch Motor oder Handkurbel angetriebenen Quetschwalze auf die andere, von dem Kontrastmaterial und der Prüfbahn umschlungenen Quetschwalze. Zweckmäßig ist dabei die angetriebene Quetschwalze ortsfest gelagert und die andere Quetschwalze gegen die angetriebene Quetschwalze anpreßbar. Der Anpreßdruck kann dabei über eine pneumatische Anpreßvorrichtung oder gegen den Druck einer Feder über einen Gewindetrieb eingestellt und an einer geeigneten Meßvorrichtung abgelesen werden.

In vielen Fällen genügt es bereits, zusammen mit der Prüfbahn ein darunterliegendes Kontrastmaterial durch die angepreßten Quetschwalzen bzw. den Farbsumpf zu führen. Um ein relativ geringes Durchschlagverhalten jedoch besser erkennbar zu machen, wird bevorzugt so verfahren, daß die Länge der Prüfbahn ein Mehrfaches der Länge des Kontrastmaterials beträgt. Vorteilhaft ist deshalb die anpreßbare Quetschwalze mit einer Lage Kontrastmaterial umwickelt. Die Länge der Testbahn beträgt dann ein Mehrfaches des Walzenumfangs.

Zum Auffangen von seitlich beim Reinigen der Vorrichtung abtropfender Beschichtungsmasse befindet sich zweckmäßigerweise unterhalb der Quetschwalzen eine Auffangwanne.

Nachfolgend wird die Erfindung an Hand eines Ausführungsbeispiels unter Hinweis auf die Zeichnungen näher erläutert. Es zeigen

Fig. 1 und 2 schematische Seitenansichten des Quetschwalzenteils der Vorrichtung in verschiedenen Ausführungsformen,

Fig. 3 eine Gesamtansicht einer Vorrichtung in perspektivischer Darstellung.

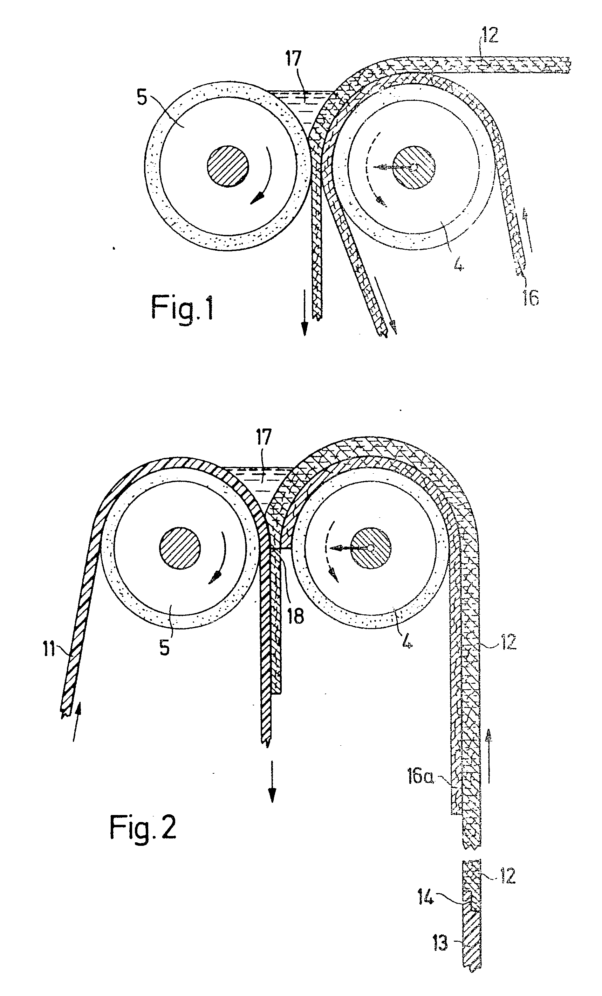

In Fig. 1 sind mit 4 und 5 die beiden gegeneinandergepreßten Quetschwalzen bezeichnet. Mit 12 ist die Prüfbahn eines Beschichtungsträgers bezeichnet, der über ein mitlaufendes Kontrastmaterial 16 durch den Farbsumpf 17 geführt wird.

Fig. 2 zeigt eine Ausführungsform, bei der an die Quetschwalze 4 ein Kontrastmaterial 16a mit seinem Anfang angeklebt ist, das in seiner Länge dem Umfang der Quetschwalze 4 entspricht. Die Anfangsmarkierung 18 der Prüfbahn 12 liegt unmittelbar vor der Berührungslinie der beiden Quetschwalzen 4, 5, das Ende der Prüfbahn 12 ist mit einer Folie als für die Beschichtungsmasse undurchdringliche Materialbahn 13 durch eine Klebnaht 14 verbunden, die gleichzeitig die Endmarkierung darstellt. Die Quetschwalze 5 ist mit einer weiteren mitlaufenden, die Beschichtungsmasse abweisenden Folienbahn 11 aus Polyäthylenfolie abgedeckt.

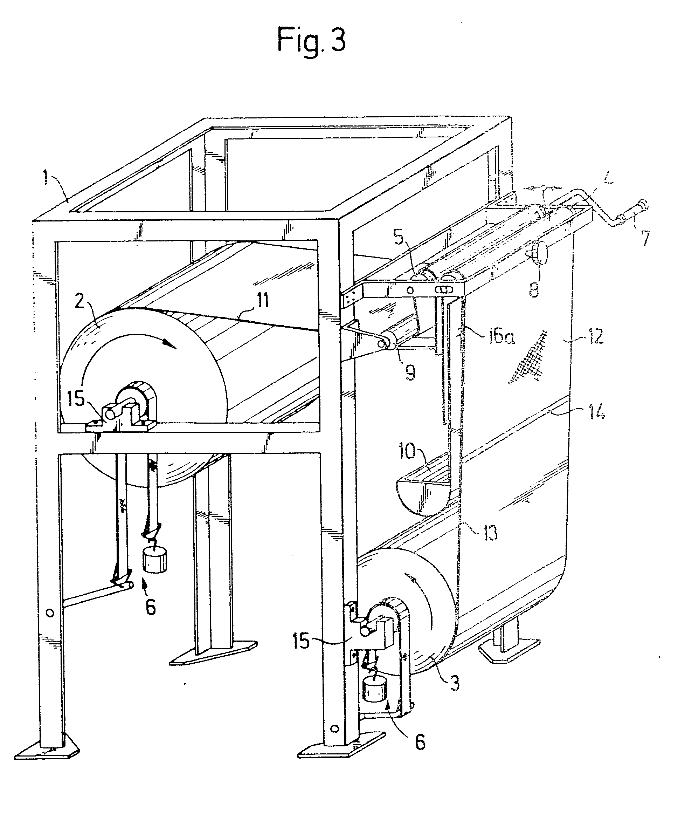

Die in Fig. 3 gezeigte Vorrichtung besteht im wesentlichen aus einem Gestell 1, an dem Lagerböcke 15 zur Aufnahme der Abwickelrollen 2 und 3 und das einen Überzug aus Gummi aufweisende Quetschwalzenpaar 4 und 5 angeordnet sind. Auf den Abwickelrollen 2, 3 befinden sich Polyäthylenfolien 11 und 13 zur Abdeckung der Quetschwalze 5 bzw. zur Verbindung mit der Prüfbahn 12. Bandbremsen 6 ermöglichen eine genaue Einstellung der Zugspannung. Die ortsfest gelagerte Quetschwalze 5 wird über die Handkurbel 7 angetrieben. Die Quetschwalze 4 ist mittels eines Drehknopfes 8 über einen nicht gezeigten Gewindetrieb gegen die Quetschwalze 5 anpreßbar und der Preßdruck kann an einer ebenfalls nicht gezeigten Skala abgelesen werden. Der Antrieb der Quetschwalze 4 erfolgt über einen nicht gezeigten Zahnradantrieb der Quetschwalze 5. Die die Quetschwalze 5 abdeckende Folienbahn 11 ist über die Umlenkwalze 9 von oben zwischen die Quetschwalzen 4, 5 geführt. Die Prüfbahn 12 ist über die Quetschwalze 4 als Leitwalze geführt. Zur besseren Erkennbarkeit sind die Folienbahn 11 und Prüfbahn 12 unterhalb des Walzenspaltes nicht in zusammengepreßtem Zustand gezeichnet worden. Die Auffangwanne 10 dient zum Auffangen von abtropfender Beschichtungsmasse und verhindert eine Beschmutzung der Abwickelrolle 3.

Beispiel

Zur Untersuchung einer weißen Papierbeschichtungsmasse wird eine in Fig. 2 und 3 gezeigte Vorrichtung verwendet. Ein als Kontrastmaterial 16a verwendetes schwarzes Papier ist, wie in Fig. 2 gezeigt, auf die Quetschwalze 4 gewickelt und an seinem Anfang mit einem nicht dargestellten Selbstklebeband befestigt. Das Kontrastmaterial 16a ist 3 cm schmaler als die Quetschwalzen 4 und 5. Als Prüfbahn 12 wird ein leichtgewichtiges Streichrohpapier von 38 g/m², das ein bekanntes Durchschlagverhalten aufweist, verwendet. Die Länge der Prüfbahn 12 entspricht dem 6fachen Umfang der Quetschwalze 4 bzw. der 6fachen Länge des Kontrastmaterials 16a. Der Anfang der Prüfbahn 12 wird zwischen die auf Spalt gestellten Quetschwalzen 4, 5 geführt. Das Ende, das gleichzeitig die Endmarkierung bildet, ist mit einer auf die Rolle 3 aufgewickelten undurchdringlichen Materialbahn 13, nämlich einer Polyäthylenfolie von 50 µm, durch eine Klebenaht 14 verbunden. Die Folienbahn 11, ebenfalls eine Polyäthylenfolie, mit gleicher Breite wie das Kontrastmaterial 16a, wird über eine Umlenkwalze 9 ebenfalls von oben in den Walzenspalt geführt. Die Quetschwalzen 4, 5 werden zusammengepreßt, wobei darauf geachtet wird, daß die Anfangsmarkierung 18 der Prüfbahn 12 genau vor der Berührungslinie der beiden Quetschwalzen 4, 5 und über dem Anfang des Kontrastmaterials 16a liegt. Die beiden Quetschwalzen 4, 5 werden zusammengepreßt und aus der zu untersuchenden Beschichtungsmasse zwischen den Quetschwalzen 4, 5 ein Farbsumpf 17 gebildet. Dann wird die Prüfbahn 12 durchgedreht, abgeschnitten und das jetzt von der Folienbahn 13 bedeckte Kontrastmaterial 16a entfernt und mit einer Bewertungsskala verglichen.

**Patentansprüche**

1. Verfahren zur Prüfung des Durchschlagverhaltens von Beschichtungsmassen und/oder für die Beschichtung vorgesehenen bandförmigen Beschichtungsträgern, bei dem ein Beschichtungsträger mit einer Beschichtungsmasse beaufschlagt wird, dadurch gekennzeichnet, daß eine Standardbeschichtungsmasse oder eine zu prüfende Beschichtungsmasse unter Anwendung von Scherung und Druck durch eine zu prüfende Bahn des Beschichtungsträgers oder durch eine ein bekanntes Durchschlagverhalten aufweisende Standardprüfbahn auf ein unter dem Beschichtungsträger angeordnetes, flächiges Kontrastmaterial gepreßt wird, das in seiner Farbe von der Beschichtungsmasse abweicht und daß die auf das Kontrastmaterial durchgedruckten Markierungen der Beschichtungsmasse als Maß für das Durchschlagverhalten der Beschichtungsmasse bzw. des Beschichtungsträgers dienen.

2. Verfahren nach Anspruch 1, dadurch gekennzeichnet, daß das flächige Kontrastmaterial ein Papier, Karton, Vliesstoff oder Folienmaterial ist.

3. Verfahren nach Anspruch 1 oder 2, dadurch gekennzeichnet, daß die eine bestimmte Testlänge des Beschichtungsträgers durchdringende Beschichtungsmasse auf einer gegenüber dieser Testlänge mehrfach verkleinerten Flächeneinheit des Kontrastmaterials aufgefangen wird.

4. Verfahren nach einem der Ansprüche 1 bis 3, dadurch gekennzeichnet, daß das Ende einer zu prüfenden Bahn eines Beschichtungsträgers oder das Ende einer ein bekanntes Durchschlagverhalten aufweisenden Standard-Prüfbahn mit einer für die Beschichtungsmasse undurchdringlichen Materialbahn verbunden ist.

5. Verfahren nach einem der Ansprüche 1 bis 4, dadurch gekennzeichnet, daß die Beurteilung des Durchschlagverhaltens durch Vergleich des für die Prüfung benutzten Kontrastmaterials mit einer Skala erfolgt, die aus Standard-Kontrastmaterial besteht, auf das Markierungen aufgebracht sind, die einem abgestuften Durchschlagverhalten entsprechen.

6. Verfahren nach einem der Ansprüche 1 bis 4, dadurch gekennzeichnet, daß die Beurteilung des Durchschlagverhaltens durch quantitative Bildanalyse, Remissionsmessung oder Farbdichtemessung erfolgt.

7. Vorrichtung für die Durchführung des Verfahrens nach einem der Ansprüche 1 bis 6, gekennzeichnet durch

a) zwei nebeneinanderliegende und gegeneinander anpreßbare Quetschwalzen (4, 5), von denen eine der Quetschwalzen (4, 5) einen Mantel aus Beschichtungsmasse abweisendem Material aufweist oder im Bereich des Walzenspaltes mit einem Beschichtungsmasse abweisenden und mitlaufenden Material (11) abgedeckt ist,

b) ein die andere der Quetschwalzen (4, 5) zumindest im Bereich des Walzenspaltes teilweise umschlingendes Kontrastmaterial (16), das in seiner Farbe von der Beschichtungsmasse abweicht.

8. Vorrichtung nach Anspruch 7, dadurch gekennzeichnet, daß zur Abdeckung der einen der Quetschwalzen (4, 5) eine für die Beschichtungsmasse abweisende Folienbahn (11) aus Kunststoff vorgesehen ist.

9. Vorrichtung nach Anspruch 8, dadurch gekennzeichnet, daß die Kunststoffolie (11) eine Polyäthylenfolie ist.

10. Vorrichtung nach einem der Ansprüche 7 bis 9, dadurch gekennzeichnet, daß eine der Quetschwalzen (4, 5) angetrieben ist und über Zahnräder oder eine Riemenverbindung die andere der Quetschwalzen (4, 5) antreibt.

11. Vorrichtung nach einem der Ansprüche 7 bis 10, dadurch gekennzeichnet, daß eine der Quetschwalzen (4, 5) ortsfest gelagert und die andere der Quetschwalzen (4, 5) gegen die eine der Quetschwalzen (4, 5) anpreßbar ist.

12. Vorrichtung nach einem der Ansprüche 7 bis 11, dadurch gekennzeichnet, daß eine der Quetschwalzen (4, 5) mit einer Lage Kontrastmaterial (16a) umwickelt ist.

13. Vorrichtung nach einem der Ansprüche 7 bis 12, dadurch gekennzeichnet, daß unterhalb der Quetschwalzen (4, 5) eine Auffangwanne (10) für die abtropfende Beschichtungsmasse angeordnet ist.

## Claims

1. Process for testing the penetration behaviour of coating compositions and/or of band-shaped coating supports provided for the coating, in which a coating composition is applied to a coating support, characterised in that a standard coating composition or a coating composition to be tested is pressed, using shearing action and pressure, through a web of the coating support to be tested or through a standard test web having a known penetration behaviour onto a sheet-like contrast material arranged underneath the coating support, the colour of the contrast material being different from that of the coating composition, and the markings made by the coating composition when it is pressed through onto the contrast material are used as a measure of the penetration behaviour of the coating composition or of the coating support.

2. Process according to claim 1, characterised in that the sheet-like contrast material is a paper, cardboard, bonded fabric or foil material.

3. Process according to claim 1 or claim 2, characterised in that the coating composition penetrating a certain test length of the coating support is collected on a unit portion of surface area of the contrast material which is several times smaller than that test length.

4. Process according to any one of claims 1 to 3, characterised in that the end of a coating support web to be tested or the end of a standard test web having a known penetration behaviour is connected to a web of material that is impermeable to the coating composition.

5. Process according to any one of claims 1 to 4, characterised in that the penetration behaviour is assessed by comparing the contrast material used for the test with a scale that consists of standard contrast material to which markings corrsponding to gradations in penetration behaviour have been applied.

6. Process according to any one of claims 1 to 4, characterised in that the penetration behaviour is assessed by quantitative picture analysis, reflectance measurement or colour density measurement.

7. Apparatus for carrying out a process according to any one of claims 1 to 6, characterised by:

a) two adjacent squeeze rollers (4, 5) that can be pressed against each other, one of which squeeze rollers (4, 5) has a jacket of a material that repels the coating composition or is covered in the region of the gap between the rollers with a material that moves along with and repels the coating composition,

b) a contrast material (16) which is partially wrapped around the orther squeeze roller (4, 5) at least in the region of the gap between the rollers, the colour of the contrast material being different from that of the coating composition.

8. Apparatus according to claim 7, characterised in that, for covering one of the squeeze rollers (4, 5), there is provided a foil web (11) of plastics material which repels the coating composition.

9. Apparatus according to claim 8, characterised in that the plastics foil (11) is a polyethylene foil.

10. Apparatus according to any one of claims 7 to 9, characterised in that one of the squeeze rollers (4, 5) is driven and drives the orther squeeze roller (4, 5) by means of toothed wheels or a belt connection.

11. Apparatus according to any one of claims 7 to 10, characterised in that one of the squeeze rollers (4, 5) is fixed in a stationary position and the other squeeze roller (4, 5) can be pressed against the first such roller (4, 5).

12. Apparatus according to any one of claims 7 to 11, characterised in that one of the squeeze rollers (4, 5) is wrapped in a layer of the contrast material (16a).

13. Apparatus according to any one of claims 7 to 12, characterised in that underneath the squeeze rollers (4, 5) there is arranged a trough (10) for collecting coating composition that drips off.

## Revendications

1. Procédé pour le contrôle de la tendance au transpercement de pâtes ou masses d'enduction et/ou de supports d'enduction en forme de bande, destinés à l'enduction ou au couchage, et dans le cas duquel une pâte d'enduction est appliquée sur un support d'enduction, caractérisé en ce qu'une pâte d'enduction ou de couchage standard, ou une pâte d'enduction ou de couchage qui doit faire l'objet d'un contrôle, est pressée, avec cisaillement et sous pression, à travers une bande du support d'enduction, laquelle doit faire l'objet d'un contrôle, ou à travers une bande d'essai standard dont la tendance au transpercement est connue, sur un matériau de contraste plan, disposé sous le support d'enduction, lequel matériau de contraste est d'une couleur différente de celle de la pâte d'enduction, et en ce que les marques imprimées, par transpercement, sur le matériau de contraste par la pâte d'enduction servent de mesure de la tendance au transpercement de la pâte ou du support d'enduction ou de couchage.

2. Procédé selon la revendication 1, caractérisé en ce que le matériau de contraste plan utilisé est un papier, un carton, un non-tissé ou un matériau en feuille mince (pellicule ou film).

3. Procédé selon l'une quelconque des revendications 1 ou 2, caractérisé en ce que la pâte d'enduction, qui traverse le support d'enduction sur une longueur d'essai déterminée de celui-ci, est recueillie sur une unité de surface du matériau de contraste qui est de plusieurs fois plus petite que cette longueur d'essai.

4. Procédé selon l'une quelconque des revendications 1 à 3, caractérisé en ce que l'extrémité d'une bande, à essayer, d'un support d'enduction, ou l'extrémité d'une bande, à essayer, d'un support d'enduction, ou l'extrémité d'une bande d'essai standard dont la tendance au transpercement est connue, est reliée à une bande de matériau qui est imperméable à la pâte d'enduction.

5. Procédé selon l'une quelconque des revendications 1 à 4, caractérisé en ce que l'évaluation de la tendance au transpercement est effectuée par une comparaison du matériau de contraste utilisé pour le contrôle avec une échelle qui est constituée par un matériau de contraste standard, sur lequel on a apposé des marques qui correspondent à un échelonnement ou une graduation de la tendance au transpercement.

6. Procédé selon l'une quelconque des revendications 1 à 4, caractérisé en ce que l'évaluation de la tendance au transpercement est effectuée par une analyse quantitative de l'image, une mesure de la luminance ou une mesure du facteur de pureté colorimétrique.

7. Dispositif pour la mise en oeuvre du procédé selon l'une quelconque des revendications 1 à 6, caractérisé par:

a) deux cylindres-presseurs (4, 5), placés l'un à côté de l'autre et qui peuvent être appuyés l'un contre l'autre, l'un de ces cylindres-presseurs (4, 5) présentant une enveloppe en un matériau qui repousse ou rejette la pâte d'enduction ou étant recouvert, dans la zone de la fente des cylindres, d'un matériau (11) qui tourne avec lui et qui rejette ou repousse la pâte d'enduction,

b) un matériau de contraste (16), dont la couleur est différente de celle de la tête d'enduction, et qui entoure partiellement, au moins dans la zone de la fente des cylindres, le second de ces cylindres-presseurs (4, 5).

8. Dispositif selon la revendication 7, caractérisé en ce que, pour recouvrir l'un des cylindres presseurs (4, 5), un lé de la feuille (11) en matière plastique, qui repousse ou rejette la pâte d'enduction, est prévu.

9. Dispositif selon la revendication 8, caractérisé en ce que la feuille de matière plastique (11) est une feuille de polyéthylène.

10. Dispositif selon l'une quelconque des revendications 7 à 9, caractérisé en ce que l'un des cylindres-presseurs (4, 5) est entraîné et entraîne le second des cylindres-presseurs (4, 5) par l'intermédiaire de roues dentées ou d'une liaison par courroie.

11. Dispositif selon l'une quelconque des revendications 7 à 10, caractérisé en ce que l'un des cylindres-presseurs (4, 5) est monté de façon fixe et que le second des cylindres-preusseurs (4, 5) peut être appuyé contre lui.

12. Dispositif selon l'une quelconque des revendications 7 à 14, caractérisé en ce que l'un des cylindres-preusseurs (4, 5) est entouré d'une couche de matériau de contraste (16a).

13. Dispositif selon l'une quelconque des revendications 7 à 12, caractérisé en de qu'une auge collectrice (10) est placée au-dessous des cylindres-preusseurs (4, 5) pour recueillir la pâte d'enduction qui tombe en s'égouttant.

Fig.1

Fig.2

# Fig. 3